# EUROPEAN PATENT APPLICATION

(11) **EP 2 229 933 A1**
(43) Date of publication of application: **22.09.2010**
(21) Application number: 08867923.8
(22) Date of filing: 26.12.2008
(51) Int. Cl.: A61K 8/41, A61K 8/34, A61K 8/42, A61K 8/49, A61Q 5/00, A61Q 5/12

(54) **HAIR COSMETIC COMPOSITION**

(30) Priority: 28.12.2007 JP 2007340021
(71) Applicant: Kao Corporation, Chuo-Ku Tokyo 103-8210 (JP)
(72) Inventor: HAYASHI, Rumi, Tokyo 131-8501 (JP); KAHARU, Takeshi, Tokyo 131-8501 (JP)
(74) Representative: HOFFMANN EITLE
(86) International application number: PCT/JP2008/003992
(87) International publication number: WO 2009/084217

(57) **Abstract**

A hair cosmetic composition is provided wherein the hair cosmetic composition contains (a) a tertiary amine represented by general formula (1) below, (b) pyrrolidonecarboxylic acid, and (c) a higher alcohol.

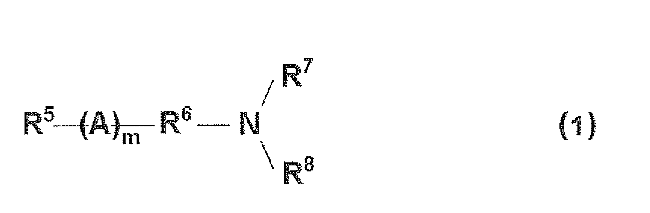

(In the formula, R⁵ represents an alkyl group having 8 to 30 carbon atoms, -A- represents an amide group or an ether group, m represents 0 or 1; when -A- is an amide group R⁶ represents an alkylene group or hydroxyalkylene group having 2 to 4 carbon atoms and when -A- is an ether group R⁶ represents an alkylene group having 2 to 4 carbon atoms; R⁷ represents a straight-chain or branched-chain alkyl group or alkenyl group having 8 to 30 carbon atoms that is optionally divided by an amide group or an ether group, or a straight-chain or branched-chain alkyl group or hydroxyalkyl group having 1 to 3 carbon atoms; and R⁸ represents a straight-chain or branched-chain alkyl group or hydroxyalkyl group having 1 to 3 carbon atoms.)

## Description

### FIELD OF THE INVENTION

The present invention relates to a hair cosmetic composition.

### BACKGROUND OF THE INVENTION

Hair cosmetic compositions such as shampoo, rinse, conditioner, treatment, hair dye, and mousse are desired to have improved hair protection and texture during use; to provide softness, smoothness, and an oily feel to hair; and to impart smoothness, ease of combing, a supple feel and so on to hair after drying.

Due to such desire, various types of quaternary ammonium salts and amine salts are formulated as cationic surfactants in conventional hair cosmetic compositions. It has been reported to employ, as a counterion for these salts, a halogen ion such as Cl⁻ (Patent Document 1), or an ion of a short chain alkylsulfuric acid such as methylsulfuric acid or ethylsulfuric acid, an ion of an organic acid such as acetic acid, tartaric acid, fumaric acid, lactic acid, malic acid, succinic acid, and a long chain alkyl group-containing carboxylic acid or sulfonic acid (Patent Documents 2 and 3). However, they do not simultaneously fully satisfy both feel during use when applying to hair and rinsing; and improvement of hair quality after drying. Furthermore, use of a specific ether amine compound together with an organic acid in order to enhance a conditioning effect has been proposed (Patent Documents 4 to 7). However, there is a desire for still further improvement in terms of better texture during use and better hair quality after rinsing.

Patent Document 1: Japanese Patent Publication No. JP-A-6-9346
Patent Document 2: International Publication WO99/11226
Patent Document 3: Japanese Patent Publication No. JP-A-9-118606
Patent Document 4: Japanese Patent Publication No. JP-A-2004-2261
Patent Document 5: Japanese Patent Publication No. JP-A-2004-67534
Patent Document 6: Japanese Patent Publication No. JP-A-2004-323495
Patent Document 7: Japanese Patent Publication No. JP-A-2007-161605

### SUMMARY OF THE INVENTION

The present invention provides a hair cosmetic composition that can provide sufficient smooth feel, softness, smoothness and, furthermore, an oily feel to hair from application through to rinsing, and can impart sufficient smoothness and ease of combing to hair after drying.

The present inventors have found that the use of a specific tertiary amine compound in combination with pyrrolidonecarboxylic acid as a cationic surfactant enables a smooth feel, softness, smoothness, and an oily feel to be imparted to hair from application through to rinsing, and softness, smoothness, and ease of combing to be imparted to hair after drying.

According to the present invention, there is provided a hair cosmetic composition containing (a) a tertiary amine represented by general formula (1) below, (b) pyrrolidonecarboxylic acid, and (c) a higher alcohol.

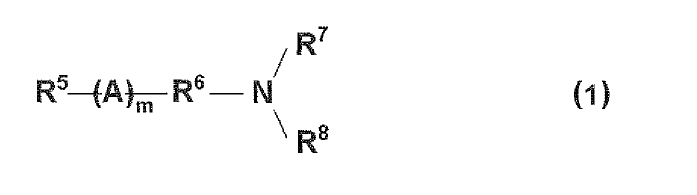

(In the formula, R⁵ represents an alkyl group having 8 to 30 carbon atoms, -A- represents an amide group or an ether group, m represents 0 or 1; when -A- is an amide group R⁶ represents an alkylene group or hydroxyalkylene group having 2 to 4 carbon atoms and when -A- is an ether group R⁶ represents an alkylene group having 2 to 4 carbon atoms; R⁷ represents a straight-chain or branched-chain alkyl group or alkenyl group having 8 to 30 carbon atoms that is optionally divided by an amide group or an ether group, or a straight-chain or branched-chain alkyl group or hydroxyalkyl group having 1 to 3 carbon atoms; and R⁸ represents a straight-chain or branched-chain alkyl group or hydroxyalkyl group having 1 to 3 carbon atoms.)

In accordance with the hair cosmetic composition of the present invention, sufficient smooth feel, softness, smoothness, and, furthermore, an oily feel can be provided to hair from application through to rinsing, and sufficient softness, smoothness, and ease of combing can be provided to hair after drying.

### DETAILED DESCRIPTION OF THE INVENTION

The hair cosmetic composition of the present invention is formed by formulating components (a) to (c) below.
(a) A tertiary amine represented by general formula (1) below,
(b) pyrrolidonecarboxylic acid, and
(c) a higher alcohol

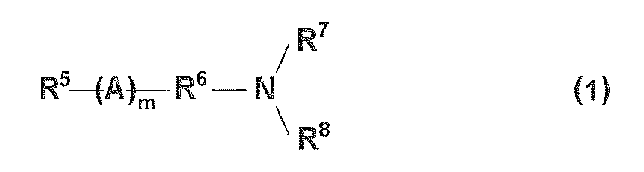

(In the formula, R⁵ represents an alkyl group having 8 to 30 carbon atoms, -A- represents an amide group or an ether group, m represents 0 or 1; when -A- is an amide group R⁶ represents an alkylene group or hydroxyalkylene group having 2 to 4 carbon atoms and when -A- is an ether group R⁶ represents an alkylene group having 2 to 4 carbon atoms; R⁷ represents a straight-chain or branched-chain alkyl group or alkenyl group having 8 to 30 carbon atoms that is optionally divided by an amide group or an ether group, or a straight-chain or branched-chain alkyl group or hydroxyalkyl group having 1 to 3 carbon atoms; and R⁸ represents a straight-chain or branched-chain alkyl group or hydroxyalkyl group having 1 to 3 carbon atoms.)

Components (a) and (b) may be present in the form of a pyrrolidonecarboxylic acid salt of the corresponding tertiary amine in the hair cosmetic composition.
The order of formulating these components in the hair cosmetic composition is not particularly limited. Specifically, component (a) and component (b) may be formulated separately to provide the hair cosmetic composition. Alternatively, component (a) and component (b) may be mixed in advance so as to form a pyrrolidonecarboxylic acid salt of the tertiary amine, and then formulated into the hair cosmetic composition.
Each of components (a) to (c) is specifically explained below.

### Component (a)

In the present invention, the tertiary amine of component (a) has a hydrophobic group, and is cationized by adding a proton in an aqueous system under acidic conditions to thus exhibit surface activity. By using component (a) represented by general formula (1) above, the feel upon use can be more stably improved from application to rinsing.

In general formula (1) above, R⁵ is preferably an alkyl group having 10 to 28 carbon atoms, and more preferably an alkyl group having 14 to 24 carbon atoms from the viewpoint of a smooth feel and an oily feel during application to hair.

In general formula (1) above, R⁷ is preferably a straight- or branched-chain alkyl or alkenyl group having 14 to 24 carbon atoms that is optionally divided by an amide group or an ether group, or a straight- or branched-chain alkyl or hydroxyalkyl group having 1 to 3 carbon atoms. R⁷ is more preferably one kind selected from the group consisting of straight- or branched-chain alkyl and hydroxyalkyl groups having 1 to 3 carbon atoms. Specifically, a methyl group, an ethyl group, and a hydroxyethyl group can be cited.
R⁸ is preferably one kind selected from the group consisting of a methyl group, an ethyl group, and a hydroxyethyl group, and is more preferably a methyl group or an ethyl group.
In addition, R⁷ and R⁸ may be the same or different groups.

In general formula (1) above, -A- represents an amide group or an ether group (namely -O-).
And m is 0 or 1, and from the viewpoint of smoothness and ease of combing after drying it is preferable that m = 1.

Moreover, from the viewpoint of further improving the balance between oily feel and softness from application to hair through to rinsing, it is preferable that in general formula (1) above m = 1 and -A- is an ether group, and in this case general formula (1) above is general formula (1-1) below.

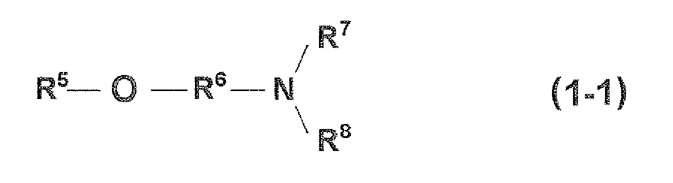

(In the formula, R⁵ to R⁸ are the same as R⁵ to R⁸ respectively in general formula (1) above.)

In general formula (1-1) above, more specifically, one in which R⁵ is an alkyl group having 14 to 24 carbon atoms, R⁶ is an alkylene group having 2 to 4 carbon atoms, and R⁷ and R⁸ are independently one kind of group selected from the group consisting of a methyl group, an ethyl group, and a hydroxyethyl group is preferable. By so doing, a smooth feel can be reliably imparted from application to hair through to rinsing.

Furthermore, m may be 1 and -A- may be an amide group, and in this case general formula (1) above is general formula (1-2) below.

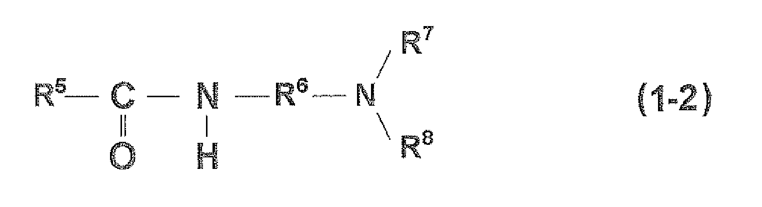

(In the formula, R⁵ to R⁸ are the same as R⁵ to R⁸ respectively in general formula (1) above.)

Furthermore, in general formula (1) above, m may be 0. In this case, general formula (1) above is general formula (1-3) below.

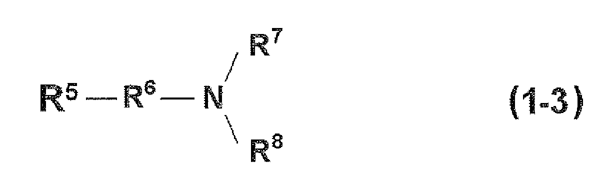

(In the formula, R⁵ to R⁸ are the same as R⁵ to R⁸ respectively in general formula (1) above.)

Specific examples of the tertiary amine represented by general formula (1) above include compounds in which m = 0 such as behenyldimethylamine and stearyldimethylamine;
compounds in which m is 1 and -A- is an ether group such as palmitoxypropyldimethylamine and stearoxypropyldimethylamine; and
compounds in which m is 1 and -A- is an amide group such as dimethylaminopropylstearamide and dimethylaminopropylbehenamide.

The hair cosmetic composition of the present invention may be one in which, as the component (a), one kind of compound is formulated or two or more kinds of compounds are formulated. For example, a compound of general formula (1) above in which -A- is an ether group and m is 1 and a compound of general formula (1) above in which -A- is an amide group and m is 1 may be used in combination.

Furthermore, the hair cosmetic composition of the present invention may further include other tertiary amine in addition to the component (a). The other tertiary amine includes a hydroxyether-based tertiary amine such as stearoxyhydroxypropyldimethylamine.

### Component (b)

In the present invention, component (b) is pyrrolidonecarboxylic acid having D- and/or L-configuration (hereinafter, also called 'PCA'). It neutralizes the tertiary amine represented by general formula (1) above, thus making the tertiary amine act as a cationic surface active agent. Pyrrolidonecarboxylic acid is also called pyroglutamic acid. Component (b) may be a DL mixture with such as D-pyrrolidonecarboxylic acid / L-pyrrolidonecarboxylic acid = 50/50, or may contain singly L-pyrrolidonecarboxylic acid or D-pyrrolidonecarboxylic acid.

### Component (c)

In the present invention, component (c) is specifically a higher alcohol having a straight- or branched-chain alkyl group or alkenyl group having 8 to 30 carbon atoms. From the viewpoint of a smooth feel and an oily feel when applying to hair, it is preferably a higher alcohol having a straight- or branched-chain alkyl or alkenyl group having 10 to 30 carbon atoms, and is more preferably a higher alcohol having a straight- or branched-chain alkyl or alkenyl group having 12 to 26 carbon atoms.

Specific examples of component (c) include cetanol (cetyl alcohol), stearyl alcohol arachidyl alcohol, behenyl alcohol, carnaubyl alcohol, and ceryl alcohol. They may be used as a mixture of more than one kind.

### Component (d)

The hair cosmetic composition of the present invention may contain a specific organic solvent (d). As the specific organic solvent, an organic solvent selected from (d1) to (d6) below may be used.
(d1): A compound represented by general formula (2) below:

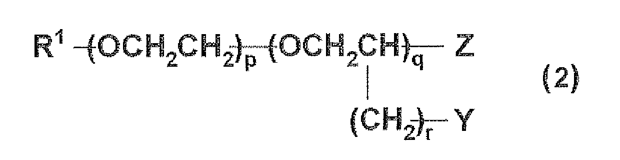

(In the formula, R¹ represents a hydrogen atom, an alkyl group having 1 to 4 carbon atoms, or a group R²¹-Ph-R²² (R²¹: a hydrogen atom, a methyl group, or a methoxy group, R²²: a direct bond or a saturated or unsaturated divalent hydrocarbon group having 1 to 3 carbon atoms, Ph: a phenylene group), Y and Z independently represent a hydrogen atom or a hydroxy group, and p, q and r independently represent an integer of 0 to 5, provided that when p = q = 0, R¹ is neither a hydrogen atom nor a group R²¹-Ph- and Z is a hydroxy group.)
(d2): An N-alkylpyrrolidone in which an alkyl group having 1 to 18 carbon atoms is bonded to the nitrogen atom.
(d3): An alkylene carbonate having an alkylene group having 2 to 4 carbon atoms.
(d4): A polypropylene glycol having a molecular weight of 100 to 5000.
(d5): A lactone or cyclic ketone represented by general formula (3), (4), or (5) below:

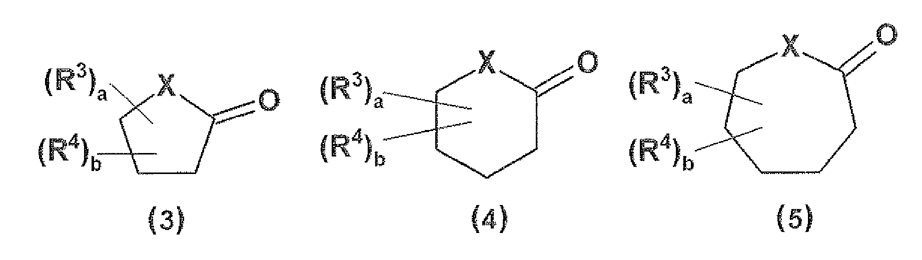

(In the formulae, X represents a methylene group or an oxygen atom, R³ and R⁴ represent mutually different substituents, and a and b independently represent 0 or 1.)

(d6): A diol represented by general formula (6) below:

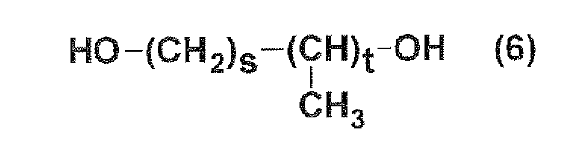

(In the formula, s and t independently represent an integer of 0 to 6.)

In the present invention, among organic solvents that are component (d), the (d1) may include ethanol, 1-propanol, 2-propanol, butanol, isobutanol, ethylene glycol, propylene glycol, benzyl alcohol, cinnamyl alcohol, phenethyl alcohol, p-anisyl alcohol, p-methylbenzyl alcohol, phenoxyethanol, 2-benzyloxyethanol, methylcarbitol, ethylcarbitol, propylcarbitol, butylcarbitol, triethylene glycol monoethyl ether, triethylene glycol monobutyl ether, and glycerol.

The (d2) may include N-methylpyrrolidone, N-octylpyrrolidone, and N-laurylpyrrolidone.

The (d3) may include ethylene carbonate and propylene carbonate.

As a polypropylene glycol of the (d4), from the viewpoint of a smooth feel and softness when rinsing after applying to hair, one having a molecular weight of 100 to 1000 is preferable.

In the (d5), with regard to R³ and R⁴ in general formulae (3) to (5) above, a straight-chain, branched-chain, or cyclic alkyl group, a hydroxy group, a sulfonic acid group, a phosphoric acid group, a carboxy group, a phenyl group, a sulfoalkyl group, an alkyl phosphate group, a carboxyalkyl group and so on is preferable. Among them a straight- or branched-chain alkyl group having 1 to 6 carbon atoms such as, for example, a methyl group, an ethyl group, a propyl group, an isopropyl group, or a butyl group is preferable, which is substituted on the γ position in the case of a γ-lactone and the δ position in the case of a δ-lactone (that is, the methylene adjacent to the heterooxygen atom).

Furthermore, when the water solubility of the compound represented by general formulae (3) to (5) is to be increased, as R³ or R⁴, an acidic group such as a sulfonic acid group, a phosphoric acid group, or a carboxy group or an alkyl group substituted with the above acidic groups is preferable.

Among the (d5), as lactones, γ-butyrolactone, γ-caprolactone, γ-valerolactone, 5-valerolactone, 5-caprolactone, δ-heptanolactone and so on can be cited, and from the viewpoint of stability of the lactone, a γ-lactone, in particular γ-butyrolactone or γ-caprolactone, is preferable.

Among the (d5), as cyclic ketones, cyclopentanone, cyclohexanone, cycloheptanone, 4-methylcycloheptanone and so on can be cited.

As the (d6), propylene glycol, 1,3-butanediol, 1,5-hexanediol and so on are preferable.

As even more preferred examples of component (d), benzyl alcohol, 2-benzyloxyethanol, propylene carbonate, propylene glycol, dipropylene glycol, and 1,3-butanediol can be cited.

Component (d) may be used in a combination of two or more kinds, and from the viewpoint of improving the feel during use, luster, and softness, the content thereof is preferably 0.01 to 50 weight % of the hair cosmetic composition of the present invention, more preferably 0.1 to 35 weight %, and particularly preferably 0.5 to 10 weight %.

### Hair cosmetic composition

The hair cosmetic composition of the present invention is formed by formulating components (a) to (c) described above. It is surmised that, due to a constitution in which pyrrolidonecarboxylic acid is used as a counterion of a tertiary amine salt and in which component (c) is contained, a bilayer membrane formed by the tertiary amine salt and the higher alcohol contributes to imparting a smooth feel, softness, smoothness, and an oily feel from application to hair through to rinsing; and softness, smoothness, and ease of combing after drying.

The hair cosmetic composition of the present invention is preferably formed by formulating components (a) to (d). Formulating component (d) enables a hair pore repair effect to be further improved, thus further improving the smoothness or ease of combing after drying.

From the viewpoint of imparting luster, softness, manageability, and suppleness to hair, the hair cosmetic composition of the present invention preferably has a pH of 2 to 5 when applied to hair (when diluted 20 times by weight with water, 25°C), more preferably 2.5 to 4, and even more preferably 3 to 4.

With regard to the hair cosmetic composition of the present invention, from the viewpoint of imparting a good texture to hair, the amount of component (a) formulated is preferably 0.1 to 20 weight %, more preferably 0.2 to 15 weight %, and even more preferably 0.5 to 10 weight %.
The amount of component (b) formulated is preferably 0.1 to 20 times by mole to component (a), more preferably 0.3 to 10 times by mole to component (a), and even more preferably 0.5 to 5 times by mole to component (a), from the viewpoint of imparting a good texture to hair by forming a salt between component (a) and component (b) and obtaining good stability by suppressing layer-separation, solidification and so on of a product.

The content of the pyrrolidonecarboxylic acid salt of the tertiary amine formed from the components (a) and (b), based on the total hair cosmetic composition, is for example at least 0.1 weight %, and preferably 0.5 weight % from the viewpoint of imparting a good texture to hair. Also, from the viewpoint of a good form for applying to hair, the content of the pyrrolidonecarboxylic acid salt of the tertiary amine, based on the total hair cosmetic composition, is for example 20 weight % or less, and preferably 15 weight % or less.

With regard to the hair cosmetic composition of the present invention, the amount of component (c) formulated is preferably 0.01 to 30 weight %, more preferably 0.5 to 25 weight %, and even more preferably 0.1 to 20 weight % in the hair cosmetic composition of the present invention from the viewpoint of a good texture of hair and emulsification/dispersion stability.

The hair cosmetic composition of the present invention preferably contains an oily component other than component (c) in order to further improve the texture provided to hair. Such an oily component may include an ester oil, an amphiphilic amide compound, a silicone, a branched fatty acid or a salt thereof and a hydrocarbon.

The ester oil optionally used in the present invention includes an ester of a fatty acid having 8 to 40 carbon atoms and a straight- or branched-chain alcohol having 1 to 4 carbon atoms, preferably an ester of a fatty acid having 10 to 24 carbon atoms and a straight- or branched-chain alcohol having 1 to 3 carbon atoms, and specifically isopropyl myristate and isopropyl palmitate.

The amphiphilic amide compound optionally used in the present invention includes one selected from diamide compounds represented by general formula (7) below.

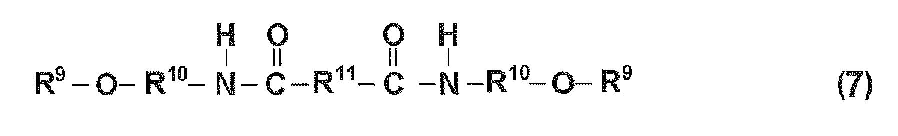

(In the formula, R⁹ represents a straight- or branched-chain hydrocarbon group having 1 to 12 carbon atoms which may be substituted with a hydroxy group and/or an alkoxy group, R¹⁰ represents a straight- or branched-chain divalent hydrocarbon group having 1 to 5 carbon atoms, and R¹¹ represents a straight- or branched-chain divalent hydrocarbon group having 1 to 22 carbon atoms.)

In the general formula (7), R⁹ is preferably a straight- or branched-chain alkyl group having 1 to 12 carbon atoms that is optionally substituted with one to three groups selected from a hydroxy group and an alkoxy group having 1 to 6 carbon atoms. Among them, an unsubstituted alkyl group having 1 to 12 carbon atoms, or an alkyl group having 2 to 12 carbon atoms that is substituted with one or two hydroxy groups, or with one alkoxy group having 1 to 6 carbon atoms, or with one hydroxy group and one alkoxy group having 1 to 6 carbon atoms is preferable.

In the formula (7), R¹⁰ is preferably a straight-or branched-chain alkylene group having 2 to 5 carbon atoms, and more preferably 2 or 3 carbon atoms. R¹¹ is preferably a straight- or branched-chain divalent hydrocarbon group having 2 to 22 carbon atoms, and more preferably a straight- or branched-chain alkylene group having 11 to 22 carbon atoms or an alkenylene group having 1 to 4 double bonds.

The diamide compound represented by the general formula (7) is more preferably a compound in which R⁹, R¹⁰, and R¹¹ are the above-mentioned preferred groups, and specific examples thereof include the compounds below.

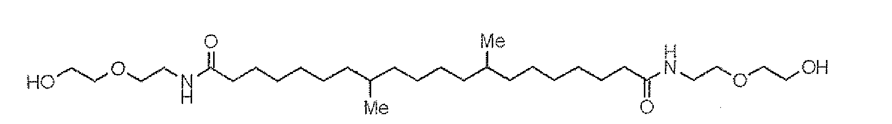

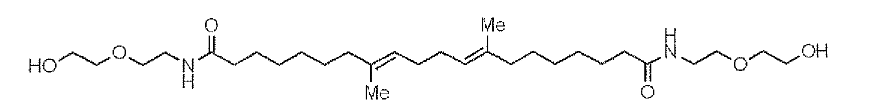

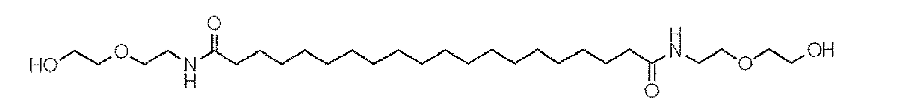

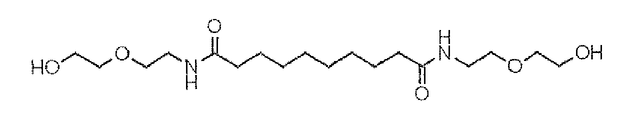

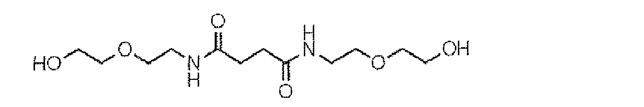

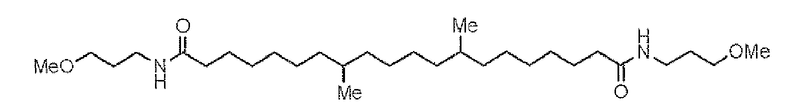

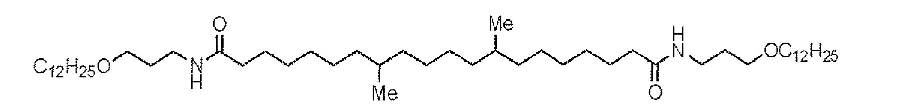

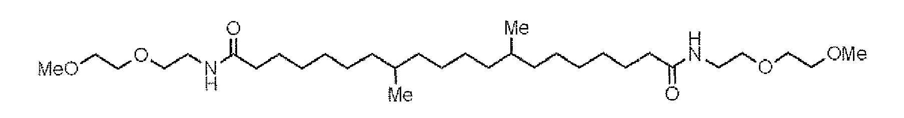

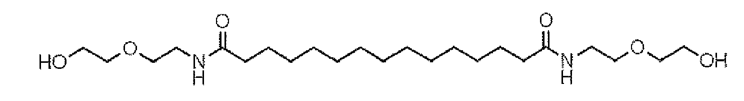

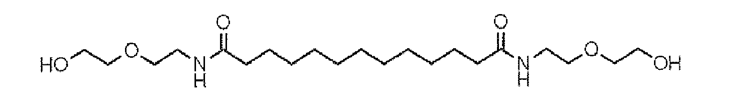

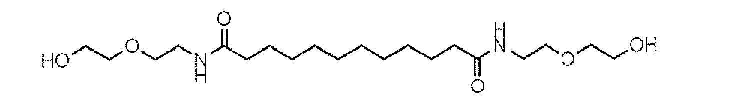

With regard to the amphiphilic amide compound, two or more kinds may be used in combination, and from the viewpoint of recovering from or suppressing damage, the content thereof is preferably 0.01 to 10 weight %, and more preferably 0.05 to 5 weight % of the hair cosmetic composition of the present invention.

The silicone optionally used in the present invention includes silicone gum, a silicone oil, a functional group-modified silicone and so on. Silicones (A) to (E) below can be exemplified. (A) Polydimethylsiloxane represented by general formula (8) below.

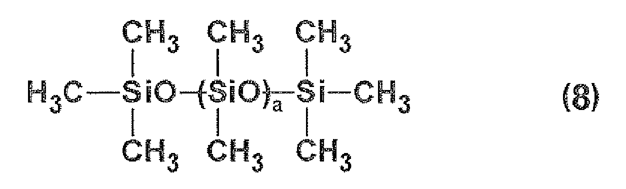

(In the formula, "a" represents an integer of 3 or greater.)
Specific examples of the polydimethylsiloxane include the SH200C series, 10cs, 50cs, 200cs, 1000cs, 5000cs, Buy11-003, BY11-026, and BY22-019 (Dow Corning Toray); the KF-96 series, 1000cs, 5000cs, KF-9008, and KF-9013 (Shin-Etsu Chemical Co., Ltd.).

When a polydimethylsiloxane of high molecular weight is formulated in the hair cosmetic composition of the present invention, it is preferable that it is formulated as a solution in a liquid oil or it is used as an emulsion or dispersion that is prepared in advance in an aqueous solution of a cationic surfactant or nonionic surfactant such as a polyoxyethylene alkyl ether. The liquid oil includes those represented by general formula (8) above in which "a" is 3 to 650, a cyclic silicone, and an isoparaffin-based hydrocarbon.

(B) Amino-modified silicone represented by general formula (9) below.

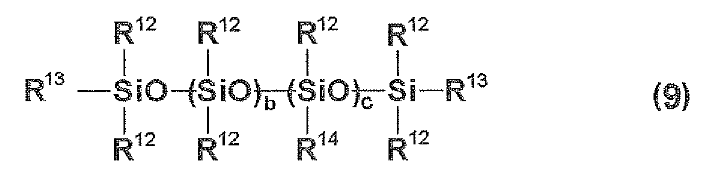

(In the formula, R¹² represents a methyl group, R¹³ represents the same group as R¹⁴, a methyl group, or a hydroxy group, R¹⁴ represents a reactive functional group represented by -R¹⁵-Q (here, R¹⁵ represents a divalent hydrocarbon group having 3 to 6 carbon atoms, and Q represents a primary to tertiary amino group-containing group or ammonium group-containing group), b and c are positive integers, and b + c depends on the molecular weight.)

In general formula (9) above, a preferred average molecular weight is 3000 to 100000.
The silicone represented by general formula (9) above includes SS-3551 and BY22-079 (Dow Corning Toray) and KF-8004 (Shin-Etsu Chemical Co., Ltd.). When the amino-modified silicone is used as an aqueous emulsion, the amount of amino-modified silicone contained in the aqueous emulsion is preferably 20 to 60 weight %, and more preferably 30 to 50 weight %.
As a preferred amino-modified silicone aqueous emulsion, SM8904 COSMETIC EMULSION (Dow Corning Toray) can be cited.

### (C) Polyether-modified silicone

The polyether-modified silicone includes, for example, SH771M, SS-2801, BY22-008, and FZ-2222 (Dow Corning Toray) and KF-6011 and KF-6017 (Shin-Etsu Chemical Co., Ltd.).
(D) Fluorine-modified silicone
(E) Alkyl-modified silicone

The branched fatty acid or a salt thereof optionally used in the present invention includes one selected from branched fatty acids represented by general formula (10) below or a salt thereof.

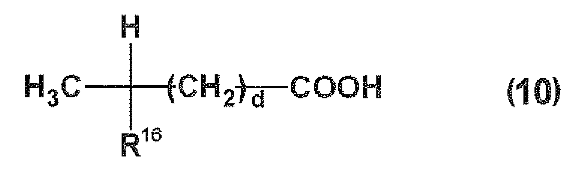

(In the formula, R¹⁶ represents a methyl group or an ethyl group, and d represents an integer of 3 to 36.)

The branched fatty acid represented by the general formula (10) may be separated and extracted from hair and so on in accordance with a method described in, for example, LIPIDS, vol. 23, No. 9, p. 878 to 881 (1988), or may be synthesized by a method described in Japanese Patent Publication No. JP-A-4-173719 or International Publication WO8/30532.

In the general formula (10), it is preferable to use one having a total number of carbon atoms of 7 to 40, more preferably 8 to 40, and even more preferably 10 to 22. Specifically, such a fatty acid includes 18-methyleicosanoic acid, 14-methylpentadecanoic acid, 14-methylhexadecanoic acid, 16-methylheptadecanoic acid, and 17-methyloctadecanoic acid.

Specific examples of the salt of a branched fatty acid represented by the general formula (10) above include an alkali metal salt such as a sodium salt, a lithium salt, or a potassium salt; an alkaline earth metal salt such as a calcium salt or a magnesium salt; an ammonium salt; an organoamine salt such as a triethanolamine salt, a diethanolamine salt, or a monoethanolamine salt; and a basic amino acid salt such as a lysine salt or an arginine salt.

The branched fatty acid may be a synthetic product or an extracted product. The extracted product includes an extract from lanolin, that is, a lanolin fatty acid and a salt thereof. Lanolin fatty acid contains about 50 weight % of methyl branched long chain fatty acids called iso-fatty acids or anteiso-fatty acids. Specifically, Crodacid 18-MEA (Croda Japan K.K.), SKLIRO (Croda Japan K.K.), and FA-NH (Nippon Fine Chemical) can be cited.

With regard to the branched fatty acid represented by general formula (10) above, two or more kinds thereof may be used in combination. Also, a synthetic product and an extracted product may be used in combination.

The branched fatty acid represented by the general formula (10) may be used as an organic acid forming a salt of the tertiary amine of component (a). It is also possible to mix the tertiary amine and the branched fatty acid in advance to thus carry out neutralization, and use as an acid adduct salt. In the hair cosmetic composition of the present invention, it is surmised that component (a) and the branched fatty acid form a hydrophobic complex.

The hydrocarbon optionally used in the present invention includes liquid isoparaffin and paraffin wax.

These oily components may be used in a combination of two or more kinds. The content of the oily component in the hair cosmetic composition of the present invention may be selected appropriately according to a good texture being provided to hair and the balance with other components, and is generally preferably 0.05 to 30 weight %, more preferably 0.1 to 25 weight %, and even more preferably 0.1 to 20 weight %.

With regard to the hair cosmetic composition of the present invention, in order to impart luster to hair and adjust the pH, an organic acid other than the above-mentioned branched fatty acid and pyrrolidonecarboxylic acid may be formulated. As such an organic acid, an organic acid having 10 or less of carbon atoms is preferable, including an acid having a short chain alkyl group having 10 or less of carbon atoms such as an alkylphosphoric acid, an alkylsulfonic acid, or an alkylsulfuric acid; an acidic amino acid such as glutamic acid or aspartic acid; an aromatic acid such as benzoic acid or p-toluenesulfonic acid; a hydroxy acid; and a dicarboxylic acid.

Among them, specific examples of the hydroxy acid include monohydroxycarboxylic acids such as glycolic acid, lactic acid, glyceric acid, gluconic acid and pantothenic acid; hydroxydicarboxylic acids such as malic acid and tartaric acid; and hydroxytricarboxylic acids such as citric acid.

Specific examples of a dicarboxylic acid include oxalic acid, malonic acid, maleic acid, succinic acid and glutaric acid.

Among them, from the viewpoint of exhibiting moisturizing and softening effects on hair, hydroxy acids, dicarboxylic acids and acidic amino acids are preferable; glycolic acid, lactic acid, malic acid, glutamic acid and aspartic acid are more preferable. It is even more preferable to contain at least one kind of organic acid selected from the group consisting of malic acid, lactic acid, glutamic acid, and glycolic acid. With regard to the hair cosmetic composition of the present invention, in particular, using pyrrolidonecarboxylic acid of component (b) and malic acid in combination enables the hair pore repair effect and luster after drying to be further improved. When PCA and malic acid are used in combination, the ratio of PCA to malic acid in the hair cosmetic composition is preferably 1:1 to 8:1, and even more preferably 2:1 to 8:1 in terms of a molar ratio, since the feel during use from application through to rinsing can be improved and the hair smoothness or the ease of combing after drying can be further improved.
In the present invention, glutamic acid may be contained in the hair cosmetic composition as an impurity of pyrrolidonecarboxylic acid.

A cationic surfactant may be formulated with the hair cosmetic composition of the present invention.
The cationic surfactant is not particularly limited as long as it is conventionally used as a raw material for cosmetics. Examples thereof include alkyltrimethylammonium chlorides such as cetyltrimethylammonium chloride,
lauryltrimethylammonium chloride and
stearyltrimethylammonium chloride;
dialkyldimethylammonium chlorides such as dicetyldimethylammonium chloride,
dilauryldimethylammonium chloride and
distearyldimethylammonium chloride; and benzalkonium chloride. One kind may be singly used, or two or more kinds may be used in combination.

Among the above-mentioned cationic surfactants, it is preferable to use an alkyltrimethylammonium chloride such as cetyltrimethylammonium chloride, lauryltrimethylammonium chloride or stearyltrimethylammonium chloride, from the viewpoint of imparting a good texture to hair.

A water-soluble polymer having an average molecular weight of 1,000 to 10,000,000 may be formulated with the hair cosmetic composition of the present invention.
Examples of such water-soluble polymers include hydroxyethylcellulose, methylcellulose, a polyoxyethylene polyoxypropylene glycol copolymer and polyethylene glycol. Among these water-soluble polymers, hydroxyethylcellulose and polyethylene glycol are preferable. These water-soluble polymers may be used in a combination of two or more kinds.

From the viewpoint of a smooth feel during rinsing after applying to hair, the content of the water-soluble polymer in the hair cosmetic composition of the present invention is preferably 0.01 to 5 weight %, more preferably 0.01 to 2 weight %, and even more preferably 0.01 to 1 weight %.

In addiction to the above-mentioned components, the hair cosmetic composition of the present invention may have appropriately a component that is conventionally used in a hair cosmetic composition depending on the intended application. Such a component includes an antidandruff agent such as dipotassium glycyrrhizinate; a vitamin; a germicide; an anti-inflammatory agent; a preservative; a chelating agent; a moisturizing agent such as sorbitol or panthenol; a coloring agent such as a dye or a pigment; a clay mineral; a pH adjusting agent such as the above-mentioned organic acid as well as an inorganic acid (hydrochloric acid, sulfuric acid, phosphoric acid and so on) or an inorganic base (sodium hydroxide, potassium hydroxide, ammonia and so on); a plant extract; a pearlescent agent; a fragrance; a UV absorber; an antioxidant; and other components described in ENCYCLOPEDIA OF SHAMPOO INGREDIENTS (Hunting, Anthony L.L., 1983, MICELLE PRESS)).

The hair cosmetic composition of the present invention is preferably a rinse-in-shampoo, a treatment, a conditioner (rinse), or a styling agent such as a blow agent, and is more preferably a rinse composition.

In accordance with the hair cosmetic composition of the present invention, it is possible to impart to hair sufficient smooth feel, softness, smoothness and, furthermore, an oily feel from application through to rinsing, and to impart sufficient smoothness and ease of combing after drying. Furthermore, the present invention can provide a hair cosmetic composition exhibiting, for example, excellent thickening properties (gel formation properties) and stability over time. It can also impart, for example, a supple feel and luster to hair after drying.

### EXAMPLES

In the Examples below, % represents weight % unless otherwise specified.
The chemical formulae of tertiary amines (a-1) to (a-4) used in the Examples below are shown in Table 1.

**[Table 1]**

| | |
|---|---|
| (a-1) | 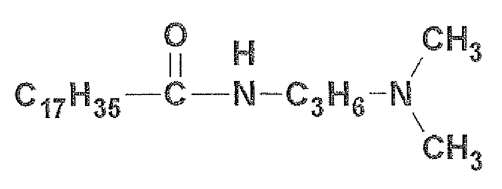 |
| (a-2) | 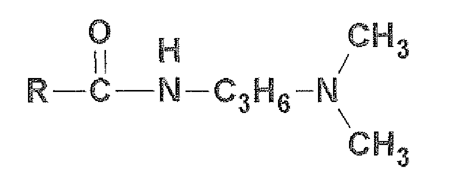 |
| (a-3) | 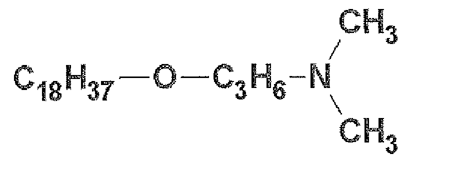 |
| (a-4) | 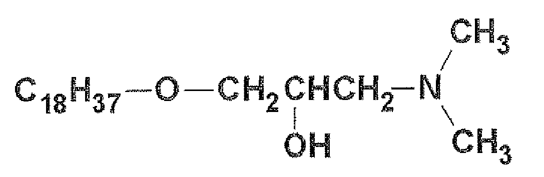 |

(a-1) NIKKOL Amidoamine MPS, manufactured by Nikko Chemicals, Co., Ltd.
(a-2) behenoylamidopropyldimethylamine, R: C₁₇H₃₅/C₁₉H₃₉/C₂₁H₄₃/C₂₃H₄₇ = 1/7/89/2%
(a-3) stearoxypropyldimethylamine, Farmin DM-E80 manufactured by Kao Corporation
(a-4) 3-octadecyloxy-2-hydroxypropyldimethylamine
Among these tertiary amines, (a-1) to (a-3) correspond to a tertiary amine represented by the general formula (1) above.

### (Examples 1 to 3 and Comparative Examples 1 to 3)

Hair rinse agents having the compositions shown in Table 2 were prepared by a standard method using (a-3) as a tertiary amine (component (a)), D-PCA and L-PCA as component (b), lactic acid, L-glutamic acid, or hydrochloric acid as a comparative acid to the component (b), stearyl alcohol as component (c), and dipropylene glycol as component (d). These hair rinse agents were evaluated by the methods below in terms of performance during application to hair, during rinsing, and after drying. The results are set forth in Table 2.

### (Evaluation methods)

20 g of Japanese female hair (length 20 cm, average diameter 80 µm) that had not been subjected to a chemical treatment such as cold permanent waving was tied in a bundle, and washed using 5 g of a shampoo. The composition of this shampoo was 15% sodium polyoxyethylene alkyl (C12) ether sulfate (average number of addition moles of ethylene oxide: 2.5), 3% diethanolamide and the balance water.
Subsequently, 2.0 g of a hair rinse agent was applied evenly, and rinsed with running water at 40°C for 30 seconds. Sensory evaluation of softness during application and rinsing, oily feel during application, smoothness during rinsing, and softness and ease of combing after drying was carried out by 10 expert panelists using the criteria below.
Excellent: 8 or more panelists evaluated that it was effective.
Good: 6 or 7 panelists evaluated that it was effective.
Fair: 3 to 5 panelists evaluated that it was effective.
Poor: 2 or less panelists evaluated that it was effective.

**[Table 2]**

| | | | | | Example | | | Comparative Example | | |
|---|---|---|---|---|---|---|---|---|---|---|
| | | | | | 1 | 2 | 3 | 1 | 2 | 3 |
| Composition of hair rinse agent | | Component (a) (%) | | (a-3) | 1.5 | 1.5 | 1.5 | 1.5 | 1.5 | 1.5 |
| | | Component (b) (molar ratio relative to Component (a)) | | D-PCA | 1 | | 0.5 | | | |
| | | | | L-PCA | | 1 | 0.5 | | | |
| | | Comparative acid (molar ratio relative to Component (a)) | | Lactic acid | | | | 1 | | |
| | | | | L-Glutamic acid | | | | | 1 | |
| | | | | Hydrochoric acid | | | | | | 1 |
| | | Component (c) (%) | | Stearyl alcohol | 5.5 | 5.5 | 5.5 | 5.5 | 5.5 | 5.5 |
| | | Component (d) (%) | | Dipropylene glycol | 3 | 3 | 3 | 3 | 3 | 3 |
| | | Polydimethylsiloxane*1 (%) | | | 2 | 2 | 2 | 2 | 2 | 2 |
| | | Citric acid(pH adjusting agent) | | | Appropriate amount | Appropriate amount | Appropriate amount | Appropriate amount | Appropriate amount | Appropriate amount |
| | | Purified water | | | Balance | Balance | Balance | Balance | Balance | Balance |
| pH | | | | | 3.5 | 3.5 | 3.5 | 3.5 | 3.5 | 3.5 |
| Evaluation results | During application | | Softness | | Excellent | Excellent | Excellent | Good | Fair | Fair |
| | | | Oily feel | | Excellent | Excellent | Excellent | Good | Fair | Poor |
| | During rinsing | | Softness | | Excellent | Excellent | Excellent | Good | Fair | Fair |
| | | | Evenness | | Good | Good | Good | Fair | Good | Fair |
| | After drying | | Softness | | Excellent | Excellent | Excellent | Good | Fair | Poor |
| | | | Ease of combing | | Good | Excellent | Excellent | Fair | Fair | Fair |

| | | | | | | | | | | |
|---|---|---|---|---|---|---|---|---|---|---|
| *1 BY11-026, manufactared by Dow Corning Toray Co., Ltd. | | | | | | | | | | |

### (Examples 4 to 8 and Reference Example 1) : Type of tertiary amine

Hair rinse agents having the compositions shown in Table 3 were produced by a standard method using the tertiary amines, acids, and components (c) and (d) shown in Table 3. Evaluation of these hair rinse agents was carried out in the same manner as in Example 1 in terms of performance during application to hair, during rinsing, and after drying. The results are set forth in Table 3.

**[Table 3]**

| | | | Example | | | | | Ref. Ex. |
|---|---|---|---|---|---|---|---|---|
| | | | 4 | 5 | 6 | 7 | 8 | 1 |
| Composition of hair rinse agent | Component (a) (%) | (a-1) | 0.8 | | | | | |
| | | (a-2) | | 1.5 | | 0.5 | 0.5 | |
| | | (a-3) | 0.8 | | 1.5 | 1 | 1 | |
| | | (a-4) | | | | | | 1.5 |
| | Acid (molar ratio relative to Component (a)) | DL-PCA | 1 | 1 | 1 | 1 | 0.8 | 1 |
| | | Malic acid | | | | | 0.2 | |
| | Component (c) (%) | Cetyl alcohol | 2 | | | | | |
| | | Stearyl alcohol | 5 | 5 | 5.5 | 5.5 | 5.5 | 5 |
| | | Behenyl alcohol | | 1 | | | | 1 |
| | Component (d) (%) | Dipropylene glycol | 3 | 3 | 3 | 3 | 3 | 3 |
| | | Benzyl alcohol | 0.3 | 0.3 | 0.3 | 0.3 | 0.3 | 0.3 |
| | Polydimethylsiloxane*1 (%) | | 2 | 2 | 2 | 2 | 2 | 2 |
| | Amino-modified silicone*2 (%) | | 0.2 | 0.2 | 0.2 | 0.2 | 0.2 | 0.2 |
| | Citric acid(pH adjusting agent) | | Appropriate amount | Appropriate amount | Appropriate amount | Appropriate amount | Appropriate amount | Appropriate amount |
| | Purified water | | Balance | Balance | Balance | Balance | Balance | Balance |
| pH | | | 3.5 | 3.5 | 3.5 | 3.5 | 3.5 | 3.5 |
| Evaluation results | During application | Softness | Excellent | Good | Excellent | Good | Excellent | Good |
| | | Evenness | Good | Excellent | Good | Excellent | Excellent | Good |
| | | Oily feel | Good | Good | Excellent | Good | Excellent | Excellent |
| | During rinsing | Softness | Excellent | Good | Excellent | Excellent | Excellent | Good |
| | | Sustainability of smoothness | Good | Excellent | Good | Excellent | Excellent | Good |
| | After drying | Smooth feel | Good | Good | Excellent | Excellent | Excellent | Good |
| | | Ease of combing | Good | Excellent | Good | Excellent | Excellent | Good |

| | | | | | | | | |
|---|---|---|---|---|---|---|---|---|
| *1 BY11-026, manufactured by Dow Corning Toray Co., Ltd. *2 SM8904 COSMETIC EMULSION, manufactured by Dow Corning Toray Co. , Ltd. | | | | | | | | |

(Examples 9 to 15 and Comparative Examples 4 to 7) :Use of organic acid in combination Hairrinse agents having the compositions shown in Table 4 were produced by a standard method using the tertiary amines, acids, and components (c) and (d) shown in Table 4. Evaluation of these hair rinse agents was carried out in the same manner as in Example 1 in terms of performance during application to hair, during rinsing, and after drying. The results are set forth in Table 4.

**[Table 4]**

| | | | Example | | | | | | | comparative Example | | | |
|---|---|---|---|---|---|---|---|---|---|---|---|---|---|
| | | | 9 | 10 | 11 | 12 | 13 | 14 | 15 | 4 | 5 | 6 | 7 |
| Composition of hair rinse agent | component (a) (%) | (a-3) | 1.7 | 1.7 | 1.7 | 1.7 | 1.7 | 1.7 | 1.7 | 1.7 | 1.7 | 1.7 | 1.7 |
| | Acid {molar ratio relative to component (a)} | DL-PCA | 1.9 | 1.6 | 1.35 | 1 | 0.75 | 1.5 | 1.5 | | | | |
| | | Malic acid | | 0.2 | 0.35 | 0.5 | 0.75 | | | 1.35 | | | 0.75 |
| | | Glutamic acid | | | | | | 1 | | | 2 | | |
| | | Lactic acid | | | | | | | 1.5 | | | 3.9 | 0.95 |
| | Component (c) (%) | Stearyl alcohol | 5.8 | 5.8 | 5.8 | 5.8 | 5.8 | 5.8 | 5.8 | 5.8 | 5.8 | 5.8 | 5.8 |
| | component (d) (%) | Dipropylene glycol | 3 | 3 | 3 | 3 | 3 | 2.5 | 3 | 3 | 2.5 | 3 | 3 |
| | | Benzyl alcohol | 0.3 | 0.3 | 0.3 | 0.3 | 0.3 | 0.3 | 0.3 | 0.3 | 0.3 | 0.3 | 0.3 |
| | Polydimethylsiloxanen^{*1} (%) | | 2 | 2 | 2 | 2 | 2 | 2 | 2 | 2 | 2 | 2 | 2 |
| | Amino-modified silicone^{*2} (%) | | 0.2 | 0.2 | 0.2 | 0.2 | 0.2 | 0.3 | 0.2 | 0.2 | 0.3 | 0.2 | 0.2 |
| | Hydroxyethylcellulose^{*3} (%) | | 0.1 | 0.1 | 0.1 | 0.1 | 0.1 | 0.1 | 0.1 | 0.1 | 0.1 | 0.1 | 0.1 |
| | Purified water | | Balance | Balance | Balance | Balance | Balance | Balance | Balance | Balance | Balance | Balance | Balance |
| pH | | | 3.4 | 3.4 | 3.4 | 3.4 | 3.4 | 3.5 | 3.4 | 3.4 | 4 | 3.4 | 3.4 |
| Evaluation results | During application | Softness | Excellent | Excellent | Excellent | Good | Good | Excellent | Excellent | Poor | Fair | Excellent | Excellent |
| | | Evenness | Good | Good | Excellent | Excellent | Excellent | Good | Good | Fair | Good | Good | Good |
| | | Oily feel | Excellent | Excellent | Excellent | Good | Good | Excellent | Excellent | Poor | Fair | Good | Poor |
| | During rinsing | Softness | Excellent | Excellent | Excellent | Good | Good | Good | Good | Poor | Good | Fair | Fair |
| | | Evenness | Good | Good | Good | Excellent | Excellent | Excellent | Good | Fair | Good | Fair | Fair |
| | | Sustainability of smoothness | Good | Excellent | Excellent | Excellent | Good | Excellent | Good | Good | Good | Fair | Good |
| | After drying | Smooth feel | Excellent | Excellent | Excellent | Good | Good | Excellent | Good | Fair | Good | Good | Good |
| | | Softness | Excellent | Excellent | Excellent | Excellent | Excellent | Good | Excellent | Poor | Good | Fair | Poor |
| | | Ease of combing | Excellent | Excellent | Excellent | Excellent | Excellent | Good | Good | Good | Fair | Good | Good |
| | | Shrine | Good | Good | Excellent | Excellent | Excellent | Excellent | Good | Excellent | Excellent | Fair | Excellent |

| | | | | | | | | | | | | | |
|---|---|---|---|---|---|---|---|---|---|---|---|---|---|
| *1 BY11-026, manufactured by Dow Corning Toray Co., Ltd. *2 SM8904 COSMETIC EMULSION, manufactured by Dow corning Toray Co., Ltd. *3 HEC Daicel SE850K, manufactured by Daicel Chemical Industries, Ltd. | | | | | | | | | | | | | |

(Examples 16 to 21 and Comparative Examples 8 to 10) : Use of organic acid in combination

Hair rinse agents having the compositions shown in Table 5 were produced by a standard method using the tertiary amines, acids, and components (c) and (d) shown in Table 5.

Evaluation of these hair rinse agents was carried out in the same manner as in Example 1 in terms of performance during application to hair, during rinsing, and after drying. The results are set forth in Table 5.

**[Table 5]**

| | | | Example | | | | | | Comparative Example | | |
|---|---|---|---|---|---|---|---|---|---|---|---|
| | | | 16 | 17 | 18 | 19 | 20 | 21 | 8 | 9 | 10 |
| Composition of hair rinse agent | Component (a) (%) | (a-2) | 1.5 | 1.5 | 1.5 | 1.5 | 1.5 | 1.5 | 1.5 | 1.5 | 1.5 |
| | Acid (molar ratio relative to Component (a)) | DL-PCA | 1.9 | 1.6 | 1.35 | 1 | 1.5 | 1.5 | | | |
| | | Malic acid | | 0.2 | 0.35 | 0.5 | | | 1.35 | | |
| | | Glutamic acid | | | | | 1 | | | 2 | |
| | | Lactic acid | | | | | | 1.5 | | | 3.9 |
| | Component (c) (%) | Stearyl alcohol | 5 | 5 | 5 | 5 | 5 | 5 | 5 | 5 | 5 |
| | Component (d) (%) | Dipropylene glycol | 2.5 | 2.5 | 2.5 | 2.5 | 2.5 | 2.5 | 2.5 | 2.5 | 2.5 |
| | | Benzyl alcohol | 0.3 | 0.3 | 0.3 | 0.3 | 0.3 | 0.3 | 0.3 | 0.3 | 0.3 |
| | Polydimethylsiloxane_{*1} (%) | | 2 | 2 | 2 | 2 | 2 | 2 | 2 | 2 | 2 |
| | Amino-modified silicone_{*2}(%) | | 0.3 | 0.3 | 0.3 | 0.3 | 0.3 | 0.3 | 0.3 | 0.3 | 0.3 |
| | Hydroxyethylcellulose_{*3}(%) | | 0.1 | 0.1 | 0.1 | 0.1 | 0.1 | 0.1 | 0.1 | 0.1 | 0.1 |
| | Purified water | | Balance | Balance | Balance | Balance | Balance | Balance | Balance | Balance | Balance |
| pH | | | 3.4 | 3.4 | 3.4 | 3.4 | 3.5 | 3.4 | 3.4 | 4 | 3.4 |
| Evaluation results | During application | Softness | Good | Good | Good | Good | Good | Good | Poor | Fair | Good |
| | | Evenness | Good | Good | Excellent | Excellent | Good | Good | Fair | Good | Good |
| | | Oily feel | Excellent | Excellent | Excellent | Good | Excellent | Excellent | Poor | Fair | Good |
| | During rinsing | Softness | Excellent | Good | Good | Good | Good | Good | Poor | Fair | Fair |
| | | Evenness | Good | Good | Excellent | Excellent | Excellent | Good | Good | Good | Fair |
| | | Sustainability of smoothness | Good | Excellent | Excellent | Excellent | Excellent | Good | Good | Good | Fair |
| | After drying | Smooth feel | Excellent | Excellent | Excellent | Good | Excellent | Good | Fair | Good | Good |
| | | Softness | Excellent | Excellent | Good | Good | Good | Excellent | Poor | Good | Fair |
| | | Ease of combing | Excellent | Excellent | Excellent | Excellent | Good | Good | Good | Fair | Good |
| | | Shine | Good | Good | Excellent | Excellent | Excellent | Good | Excellent | Excellent | Fair |

| | | | | | | | | | | | |
|---|---|---|---|---|---|---|---|---|---|---|---|
| *1 BY11-026, manufactured by Dow Corning Toray Co., Ltd. *2 SM8904 COSMETIC EMULSION, manufactured by Dow corning Toray Co., Ltd. *3 HEC Daicel SE850K, manufactured by Daicel Chemical Industries, Ltd. | | | | | | | | | | | |

All of the hair rinse agents of Examples 1 to 21 had a good texture during application, and sufficient thickening properties (gel formation properties). Furthermore, they gave a supple feel and good luster after drying.
Moreover, when the increase in viscosity was examined for the hair rinse agents of Examples 1 to 21 after storing at room temperature (25°C) for 1 month, there was no marked increase in viscosity that would be inconvenient for use.

Using an ether amine (a-3) as component (a) enabled the balance between an oily feel and softness from application to hair through to rinsing to be further improved compared with cases in which amide amines (a-1) and (a-2) were used.
Furthermore, using (a-3) as an ether amine type tertiary amine enabled the softness from application to hair through to rinsing to be further improved compared with a case in which a hydroxyether amine (a-4) was used.

### (Example 22)

A hair conditioner having the composition below was produced. This hair conditioner imparted softness, smoothness, and an oily feel during application and during rinsing, and the hair after drying had sufficient smoothness and luster and ease of combing.

**Composition**

| | |
|---|---|
| (a-3) | 1.50% |
| (a-2) | 0.25% |
| DL-PCA^{*1} | 1.05% |
| Glycolic acid | 0.20% |
| Stearyl alcohol | 5.50% |
| Benzyl alcohol | 0.50% |
| Dipropylene glycol | 2.50% |
| Cholesteryl isostearate | 0.10% |
| Hydroxyethylcellulose^{*2} | 0.10% |
| Polydimethylsiloxane^{*3} | 2.00% |
| Polydimethylsiloxane^{*4} | 2.00% |
| Amino-modified silicone^{*5} | 0.50% |
| Fragrance | appropriate amount |
| Purified water | Balance |
| (pH 3.3) | |

| | |
|---|---|
| *1 AJIDEW A-100, manufactured by Ajinomoto Co., Inc. *2 HEC Daicel SE850K, manufactured by Daicel Chemical Industries, Ltd. *3 BY11-040, manufactured by Dow Corning Toray *4 BY11-026, manufactured by Dow Corning Toray *5 SM8904 COSMETIC EMULSION, manufactured by Dow Corning Toray | |

### (Example 23)

A hair conditioner with an antidandruff effect having the composition below was produced. This hair conditioner imparted softness, smoothness, and an oily feel during application and during rinsing, the hair after drying had sufficient smoothness and ease of combing, and an antidandruff effect could be confirmed.

**Composition**

| | |
|---|---|
| (a-3) | 1.50% |
| DL-PCA^{*1} | 1.00% |
| Malic acid | 0.50% |
| Stearyl alcohol | 4.00% |
| Dipotassium glycyrrhizinate^{*2} | 0.10% |
| Benzyloxyethanol | 1.00% |
| Isopropyl palmitate | 0.50% |
| Hybrid sunflower oil | 0.50% |
| Hydroxyethylcellulose^{*3} | 0.30% |
| Polydimethylsiloxane^{*4} | 3.00% |
| Amino-modified silicone^{*5} | 0.20% |
| Fragrance | appropriate amount |
| Purified water | Balance |
| (pH 3.7) | |

| | |
|---|---|
| *1 AJIDEW A-100, manufactured by Ajinomoto Co., Inc. *2 Glytinon K2, manufactured by Tokiwa Phytochemical Co., Ltd. *3 HEC Daicel SE850K, manufactured by Daicel Chemical Industries, Ltd. *4 BY11-040, manufactured by Dow Corning Toray *5 SM8904 COSMETIC EMULSION, manufactured by Dow Corning Toray | |

### (Example 24)

A hair treatment having the composition below was produced. This hair treatment imparted softness, smoothness, and an oily feel during application and during rinsing, and the hair after drying had sufficient smoothness and ease of combing.

**Composition**

| | |
|---|---|
| (a-3) | 2.50% |
| DL-PCA^{*1} | 1.30% |
| Malic acid | 0.20% |
| Lactic acid | 0.20% |
| Stearyl alcohol | 8.00% |
| Benzyl alcohol | 1.00% |
| Dipropylene glycol | 5.00% |
| 18-Methyleicosanoic acid^{*2} | 0.10% |
| Dipentaerythritol fatty acid ester | 0.30% |
| Polypropylene glycol^{*3} | 0.20% |
| Polydimethylsiloxane^{*4} | 3.00% |
| Polydimethylsiloxane^{*5} | 2.00% |
| Amino-modified silicone^{*6} | 0.50% |
| (Bisisobutyl PEG-15/amodimethicone) copolymer^{*7} | 0.20% |
| Fragrance | appropriate amount |
| Purified water | Balance |
| (pH 3.3) | |

| | |
|---|---|
| *1 AJIDEW A-100, manufactured by Ajinomoto Co., Inc. *2 Crodacid 18MEA, manufactured by Croda Japan K.K. *3 POLYOX WSR N-60K, manufactured by The Dow Chemical Company *4 BY11-040, manufactured by Dow Corning Toray *5 BY11-026, manufactured by Dow Corning Toray *6 SM8904 COSMETIC EMULSION, manufactured by Dow Corning Toray *7 DOW CORNING TORAY SS-3588, manufactured by Dow Corning Toray | |

In the Examples above, a specific amount of component (a) was formulated in the hair cosmetic composition, but even when the amount of component (a) formulated is in the range of, for example, 0.1 to 20 weight % relative to the total hair cosmetic composition, a hair cosmetic composition having an excellent feel in use during application and rinsing and after drying can be obtained.
Also, in the Examples above, the ratio of component (b) and component (a) formulated was set at a specific value, but even when the amount of component (b) formulated is in the range of, for example, 0.1 to 20 times by mole relative to component (a), a hair cosmetic composition having an excellent feel in use during application and rinsing and after drying can be obtained.

## Claims

1. A hair cosmetic composition comprising:
(a) a tertiary amine represented by general formula (1) below;
(b) pyrrolidonecarboxylic acid; and
(c) a higher alcohol, 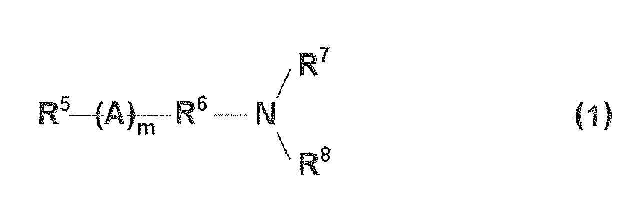 wherein R⁵ represents an alkyl group having 8 to 30 carbon atoms, -A- represents an amide group or an ether group, m represents 0 or 1; when -A- is an amide group R⁶ represents an alkylene group or hydroxyalkylene group having 2 to 4 carbon atoms and when -A- is an ether group R⁶ represents an alkylene group having 2 to 4 carbon atoms; R⁷ represents a straight-chain or branched-chain alkyl group or alkenyl group having 8 to 30 carbon atoms that is optionally divided by an amide group or an ether group, or a straight-chain or branched-chain alkyl group or hydroxyalkyl group having 1 to 3 carbon atoms; and R⁸ represents a straight-chain or branched-chain alkyl group or hydroxyalkyl group having 1 to 3 carbon atoms.

2. The hair cosmetic composition as set forth in Claim 1,
wherein said tertiary amine (a) is a combination of a compound represented by general formula (1) above in which -A- is an ether group and m is 1 and a compound represented by general formula (1) above in which -A- is an amide group and m is 1.

3. The hair cosmetic composition as set forth in Claim 1 or 2, further comprising
(d): an organic solvent selected from the group consisting of (d1) to (d6) below;
(d1): a compound represented by general formula (2) below, 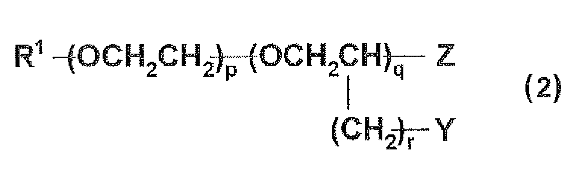 wherein R¹ represents a hydrogen atom, an alkyl group having 1 to 4 carbon atoms, or a group R²¹-Ph-R²² (R²¹:
a hydrogen atom, a methyl group, or a methoxy group;
R²²: a direct bond or a saturated or unsaturated divalent hydrocarbon group having 1 to 3 carbon atoms;
Ph: a phenylene group), Y and Z independently represent a hydrogen atom or a hydroxy group, and p, q and r independently represent an integer of 0 to 5,
provided that when p = q = 0, R¹ is neither a hydrogen atom nor a group R²¹-Ph- and Z is a hydroxy group;
(d2): an N-alkylpyrrolidone in which an alkyl group having 1 to 18 carbon atoms is bonded to the nitrogen atom;
(d3): an alkylene carbonate having an alkylene group with 2 to 4 carbon atoms;
(d4): a polypropylene glycol having a molecular weight of 100 to 5000;
(d5): a lactone or cyclic ketone represented by general formula (3), (4) or (5) below, 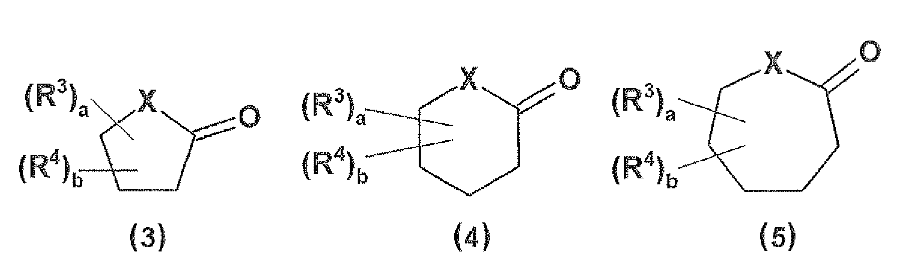 wherein X represents a methylene group or an oxygen atom, R³ and R⁴ represent mutually different substituents, and a and b independently represent 0 or
1;
(d6): a diol represented by general formula (6) below, 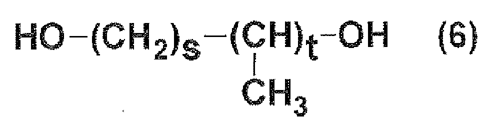 wherein s and t independently represent an integer of 0 to 6.

4. The hair cosmetic composition as set forth in any of Claims 1 to 3, further comprising at least one or more kinds of organic acid selected from the group consisting of malic acid, lactic acid, glutamic acid and glycolic acid.

5. The hair cosmetic composition as set forth in any of Claims 1 to 4,
wherein the hair cosmetic composition has a pH of 2 to 5 at 25 °C when diluted 20 times by weight with water.
